# EUROPÄISCHE PATENTSCHRIFT

(11) **EP 1 784 137 B1**
(45) Veröffentlichungstag und Bekanntmachung des Hinweises auf die Patenterteilung: **02.09.2015**
(21) Anmeldenummer: 05773708.2
(22) Anmeldetag: 12.07.2005
(51) Int. Cl.: A61B 18/14

(54) **CHIRURGISCHES INSTRUMENT**
SURGICAL INSTRUMENT
INSTRUMENT CHIRURGICAL

(30) Priorität: 24.08.2004 DE 102004040959
(43) Veröffentlichungstag der Anmeldung: 16.05.2007
(73) Patentinhaber: ERBE Elektromedizin GmbH, 72072 Tübingen (DE)
(72) Erfinder: BRODBECK, Achim, 72555 Metzingen (DE); HAFNER, Dieter, 72072 Tübingen (DE)
(74) Vertreter: Bohnenberger, Johannes
(86) Internationale Anmeldenummer: PCT/EP2005/007559
(87) Internationale Veröffentlichungsnummer: WO 2006/021269

(56) Entgegenhaltungen:
- EP-A- 0 724 863
- US-A- 5 445 638
- US-A- 5 458 598
- US-A1- 2002 099 371
- US-A1- 2003 125 728
- US-A1- 2004 116 924
- US-B1- 6 558 385

## Beschreibung

Die Erfindung betrifft ein chirurgisches Instrument nach dem Oberbegriff des Patentanspruches 1.

Derartige Instrumente werden zum Schneiden, Präparieren und Koagulieren von Gewebe insbesondere in der minimalinvasiven Chirurgie, z.B. der Laparoskopie eingesetzt. Eine wesentliche Anforderung an derartige Geräte besteht darin, dass Gewebe in einfacher Weise sicher gegriffen werden kann und das Gewebe dann ebenfalls sicher durchtrennt werden kann. Dabei ist es auch wichtig, dass durch das Durchtrennen des Gewebes und die dazu notwendige Schneidbewegung nicht die Gefahr besteht, dass umgebendes Gewebe verletzt wird.

Aus der US 5,445,638 ist ein derartiges Instrument bekannt, das als Rohrschaftinstrument ausgebildet ist und Elektroden aufweist, um das erfasste Gewebe zu koagulieren.

Als problematisch wird bei diesem Instrument angesehen, dass einerseits ein hoher Verschleiß am Schneidinstrument auftritt, andererseits das Gewebe beim Durchtrennen relativ leicht aus den Klemmflächen herausrutscht und somit nicht vollständig durchtrennt wird.

Der Erfindung liegt die Aufgabe zu Grunde, ein chirurgisches Instrument der eingangs genannten Art dahin gehend weiter zu bilden, dass eine verbesserte Schneidfunktion bei guter und sicherer Bedienbarkeit dauerhaft sichergestellt wird.

Diese Aufgabe wird bei einem chirurgischen Instrument insbesondere für laparoskopische oder dergleichen minimalinvasive Eingriffe mit mindestens zwei gegeneinander bewegbaren Klemmteilen mit Klemmflächen zum Fassen von Gewebe in einem Schließzustand der Klemmflächen und einer Schneideinrichtung mit einer Schneidkante, die in einer Schneidrichtung relativ zu den Klemmteilen zum Schneiden des gefassten Gewebes bewegbar ist, dadurch gelöst, dass die Schneideinrichtung im Schließzustand in einem spitzen Winkel relativ zu den Klemmflächen verläuft. Anspruch 1 definiert die Erfindung. Dokument US 2002/0099371 offenbart ein instrument gamäß dem Oberbegriff von Anspruch 1.

Ein wesentlicher Punkt der Erfindung liegt somit darin, dass die Klemmflächen das Gewebe nicht - wie beim eingangs genannten Stand der Technik - so halten, dass das Schneidinstrument ausschließlich Kräfte auf das Gewebe ausübt, die es aus den Klemmflächen herausschieben. Vielmehr wird durch die winklige Anordnung erreicht, dass zumindest Teile der aufgebrachten Schneidkraft senkrecht zu den Klemmflächen wirken und das Gewebe somit eher noch sicherer im Griff der Klemmflächen bleibt. Dabei bleibt dennoch eine gute Bedienbarkeit des Instrumentes sichergestellt, da das Instrument so angeordnet werden kann, dass bei geöffneten Klemmteilen ein Vorschieben zum Ergreifen von Gewebe im Wesentlichen in Richtung einer Winkelhalbierenden zwischen den Klemmflächen geschehen kann, was insbesondere bei laparoskopischen Operationen von großem Vorteil ist.

Ein weiterer, wesentlicher Vorteil des Instrumentes liegt darin, dass die Schneidkante des Schneidinstrumentes bei ihrem Weg durch den Spalt zwischen den Klemmflächen nicht konstant mit demselben Abschnitt auf das Gewebe trifft, sondern dieser Abschnitt über die Schneidkante hinweg wandert, so dass einerseits eher geschnitten als gedrückt und andererseits die Schneidkante über einen größeren Längenabschnitt hinweg benutzt und somit weniger abgenutzt wird.

Die Schneideinrichtung kann als HF-chirurgische Schneideinrichtung oder aber als mechanische Schneideinrichtung (skalpellartig) ausgebildet sein.

Vorzugsweise ist die Schneidkante in einem stumpfen Winkel relativ zur Schneidrichtung geneigt, was die eigentliche Schneidwirkung verbessert. Darüber hinaus wird der Weg, über den die Schneideinrichtung verschoben werden muss, verringert. Dieser Neigungswinkel relativ zur Schneidrichtung kann über die Länge der Schneidkante konstant sein oder sich bei einer bogenförmig geschwungenen Schneidkante über deren Länge hin verändern. Bei einer solchen bogenförmigen Schneidkante kann ein "sanfter" Anschnitt geschehen, der dann in einen steileren, kräftiger schneidenden Schnittverlauf übergeht.

Vorzugsweise ist eines der Klemmteile relativ zur Schneidrichtung unbeweglich. Dadurch hat die Bedienungsperson einen festen Bezugspunkt beim Ergreifen von Gewebe. Darüber hinaus ist die Mechanik zum Bewegen der Klemmteile einfach.

Vorzugsweise ist das chirurgische Instrument an einem distalen Ende eines Halters zur Bildung eines Rohrschaftinstrumentes für laparoskopische oder dergleichen minimalinvasive Eingriffe befestigt. Das so entstehende Instrument ist gut handhabbar. Insbesondere ist Gewebe aufgrund der Schrägstellung gegenüber einer Achse des Rohrschaftes leicht zu ergreifen und zu präparieren.

Die Klemmfläche weist vorzugsweise in an sich bekannter Weise Elektroden zum Zuführen eines HF-Koagulationsstromes zum Gewebe auf, so dass mit den Klemmflächen ergriffenes Gewebe koaguliert und dann durchtrennt werden kann.

Nachfolgend werden Ausführungsbeispiele der Erfindung anhand von Abbildungen näher erläutert. Hierbei zeigen.
- - Fig. 1: eine perspektivische Ansicht einer ersten Ausführungsform der Erfindung,
- - Fig. 2: eine perspektivische Ansicht der Ausführungsform nach Fig. 1 aus einem anderen Blickwinkel,
- - Fig. 3: eine perspektivische Darstellung einer Schneideinrichtung,
- - Fig. 4: eine perspektivische Darstellung der Anordnung nach Fig. 1 mit geschlossenen Klemmteilen,
- - Fig. 5: eine Seitenansicht der Ausführungsform nach Fig. 4,
- - Fig. 6: eine Darstellung der Ausführungsform mit nur einem Klemmteil,
- - Fig. 7: ein zweites Klemmteil zum Zusammenbau mit dem Klemmteil nach Fig. 6,
- - Fig. 8: einen Schnitt entlang der Ebene VIII-VIII aus Fig. 4,
- - Fig. 9: eine schematisierte Schnittdarstellung zur Erläuterung der verschiedenen Winkel,
- - Fig. 10: eine modifizierte Ausführungsform der Anordnung nach Fig. 9 mit einer schräg gestellten Schneidkante und
- - Fig. 11: eine Darstellung entsprechend der nach den Fig. 9 oder 10 mit einer bogenförmigen Schneidkante.

In der nachfolgenden Beschreibung werden für gleiche und gleich wirkende Teile dieselben Bezugsziffern verwendet.

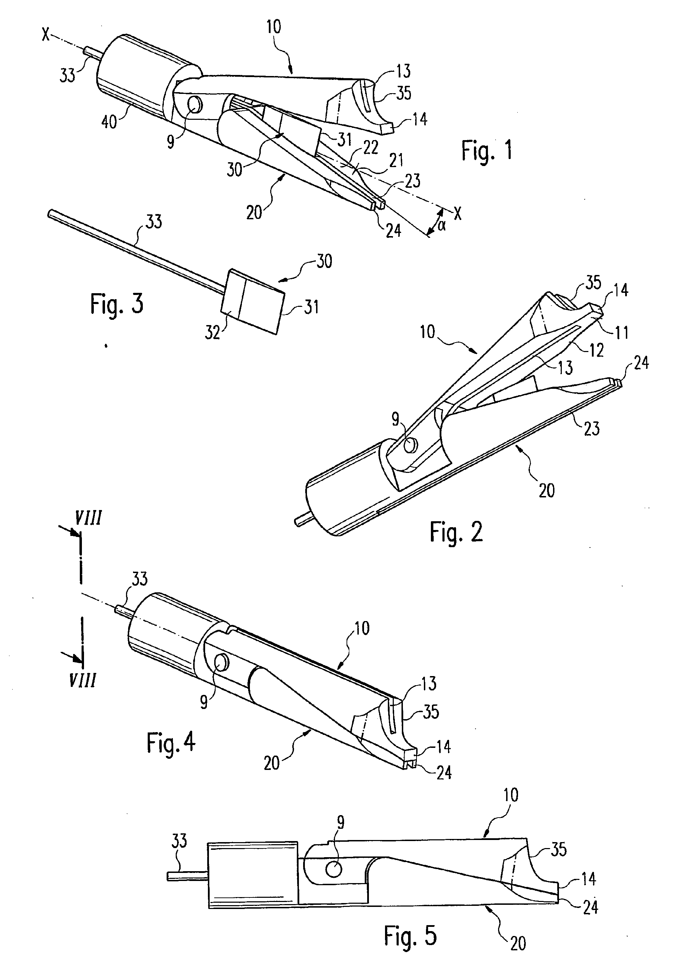

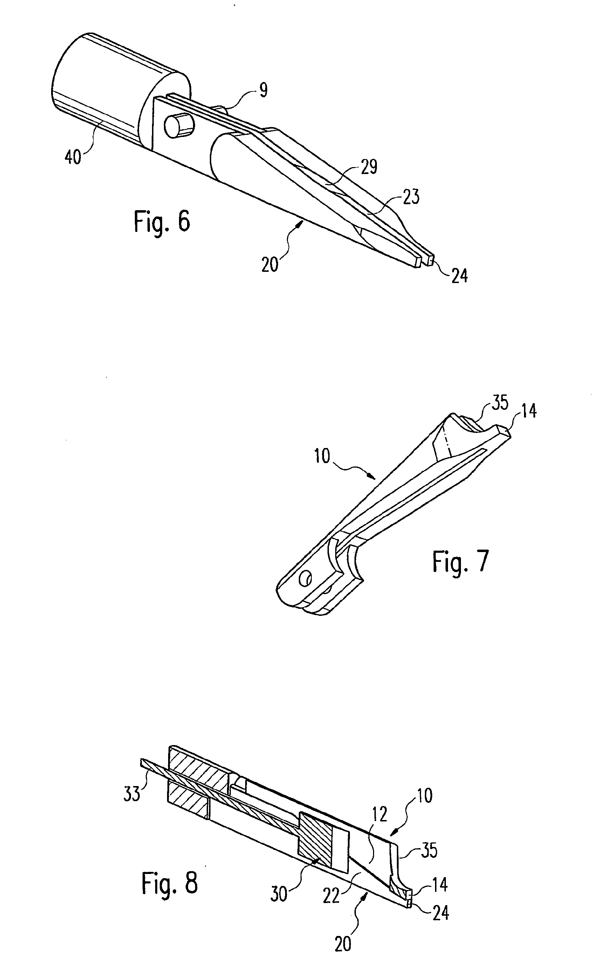

Wie aus den Abbildungen 1 - 8 hervorgeht umfasst das chirurgische Instrument ein erstes, in den Zeichnungen oberes Klemmteil 10 und ein zweites, in den Zeichnungen unteres Klemmteil 20. Jedes der Klemmteile 10 und 20 weist eine Klemmfläche 11 bzw. 21 auf, die in an sich bekannter Weise mit Elektroden 12 bzw. 22 versehen sind. Die Klemmteile 10 und 20 sind über ein Gelenk 9 miteinander verbunden, wobei das (obere) Klemmteil 10 eine Betätigungseinrichtung aufweist, die an sich bekannt ist und in den Abbildungen der Einfachheit halber nicht gezeigt wurde.

Wenn zwischen den beiden Klemmflächen 11 und 21 Gewebe eingeklemmt ist, so kann über die Elektroden 12, 22 ein HF-Strom zur Koagulation des Gewebes in dieses eingeleitet werden.

Das obere Klemmteil 10 und das untere Klemmteil 20 weisen jeweils einen Schlitz 13 bzw. 23 auf, in welchem eine Schneideinrichtung 30 mit einer Schneidfläche 32 und einer vorderen Schneidkante 31 über eine Schubstange 33 hin und her bewegbar ist.

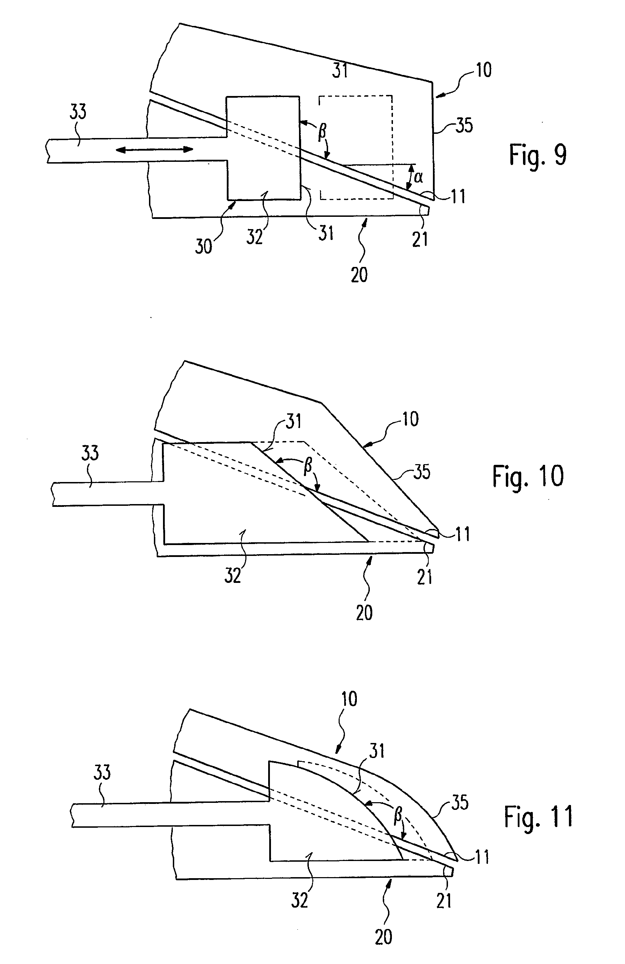

Die Klemmflächen 11, 21 weisen gegenüber einer Längsachse X, in welcher die Schneideinrichtung 30 über ihre Schubstange 33 vor und zurück bewegt werden kann, im geschlossenen Zustand (Fig. 4/5) einen Winkel α auf (siehe Fig. 1, 9). Dieser spitze Winkel entspricht im Wesentlichen bei der in Fig. 1 - 8 gezeigten Ausführungsform der Winkelhalbierenden zwischen den beiden Klemmflächen 11, 21 in deren geöffnetem Zustand (Fig. 1). Das untere Klemmteil 20 ist an einem Halter 40 starr befestigt, der zu einem Rohrschaftinstrument gehört, wie dieses allgemein bekannt ist. Durch die so getroffene Anordnung ist es möglich, das Rohrschaftinstrument in seiner Bewegungsrichtung entlang der Achse X auf ein zu fassendes Gewebeteil zuzubewegen und dieses zu ergreifen. Diese Bewegungsrichtung ist für die handhabende Person besonders günstig.

Die Spitze 14 des oberen und die Spitze 24 des unteren Klemmteils sind jeweils schlank ausgebildet, so dass auch kleinere Gewebeabschnitte sicher gegriffen werden können.

Bei der in den Fig. 1 - 8 gezeigten Ausführungsform der Erfindung tritt die Schneideinrichtung 30 mit ihrer Schneidkante 31 aus dem Schlitz 13 des oberen Klemmteils 10 dann aus, wenn die Schneidkante 31 in ihrer vordersten (distalen) Position angelangt ist.

Um hier einen Schutz vor Verletzungen umgebenden Gewebes zu erreichen, ist bei der Ausführungsform nach Fig. 9 ein Vorderrand 35 des oberen Klemmteils 10 so weit nach vorne gezogen, dass die Schneidkante 31 in der vorderen Extremposition der Schneideinrichtung 30 vollständig geschützt ist.

Bei der Ausführungsform nach Fig. 10 ist der stumpfe Winkel β, um welchen die Schneidkante 31 relativ zu den Klemmflächen 11, 21 (im geschlossenen Zustand) geneigt ist, größer als bei der Ausführungsform nach Fig. 9. Wie aus den Abbildungen 9 und 10 im Vergleich ersichtlich, ist der Weg, um welchen die Schneideinrichtung 30 entlang der Achse X verschoben werden muss, um von der in den Abbildungen gezeigten mittleren Position bis zur vordersten Position zu kommen, geringer als bei der Ausführungsform nach Fig. 9 (auf die unterbrochenen Linien wird verwiesen). Darüber hinaus kann der Vorderrand 35 abgeschrägt werden, ohne dass die Schneidkante 31 in der vordersten Position der Schneideinrichtung 30 aus dem Instrument austreten würde. Dadurch ergibt sich eine schlankere Bauform.

Die in Fig. 11 gezeigte Ausführungsform der Erfindung unterscheidet sich von der nach Fig. 10 dadurch, dass die Schneidkante 31 nicht gleichmäßig schräg gestellt sondern gewölbt ausgeführt ist. Dadurch kann einerseits das obere Klemmteil 10 noch schlanker ausgebildet werden, andererseits ergibt sich ein "sanfterer" Anschnitt, wenn die Schneideinrichtung 32 von ihrer zurückgezogenen Position (in den Abbildungen nach links gezogene Position) in ihre vorgeschobene Position verschoben wird und dabei der Winkel zwischen einem eingeklemmten Gewebeteil zuerst sehr flach, fast parallel zu den Klemmflächen 11, 21 und dann immer steiler werdend zu diesen variiert.

Darüber hinaus ist es auch möglich, die Klemmflächen 11, 21 nicht geradlinig sondern ebenfalls gekrümmt auszubilden, was für manche Präparationszwecke besonders vorteilhaft ist.

### Bezugszeichenliste

- 9: Gelenk
- 10: oberes Klemmteil
- 11: obere Klemmfläche
- 12: Elektrode
- 13: Schlitz
- 14: Spitze
- 20: unteres Klemmteil
- 21: untere Klemmfläche
- 22: Elektrode
- 23: Schlitz
- 24: Spitze
- 29: Stangendurchlass
- 30: Schneideinrichtung
- 31: Schneidkante
- 32: Schneidfläche
- 33: Schubstange
- 35: Vorderrand
- 40: Halter

## Patentansprüche

1. Rohrschaftinstrument mit einem Halter (40), wobei am distalen Ende des Halters (40) ein chirurgisches Instrument befestigt ist, wobei das Instrument umfasst:
- mindestens ein erstes Klemmteil (10) und ein zweites Klemmteil (20), wobei das erste Klemmteil (10) eine erste Klemmfläche (11) und das zweite Klemmteil (20) eine zweite Klemmfläche (21) aufweist, wobei die Klemmteile (10, 20) zum Fassen von Gewebe in einem Schließzustand der Klemmflächen (11, 21) gegeneinander bewegbar sind;
- eine Schneideinrichtung (30) mit einer Schneidkante (31), die in einer Schneidrichtung relativ zu den Klemmteilen (10, 20) zum Schneiden des gefassten Gewebes bewegbar ist;
- einen ersten Schlitz (13) im ersten Klemmteil (10);
- einen zweiten Schlitz (23) im zweiten Klemmteil (20), wobei die Schneideinrichtung (40) mit der Schneidkante (31) in den Schlitzen (13) hin und her bewegbar ist;
wobei das zweite Klemmteil (20) mit der zweiten Klemmfläche (21) relativ zur Schneidrichtung unbeweglich am Halter (40) befestigt ist;
**dadurch gekennzeichnet, dass**
die genau eine erste Klemmfläche (11), die im Schließzustand in einem spitzen Winkel (α) relativ zur Schneidrichtung geneigt ist, durch den ersten Schlitz (13) und die genau ein zweite Klemmfläche (21), die im Schließzustand in einem spitzen Winkel (α) relativ zur Schneidrichtung geneigt ist, durch den zweiten Schlitz (23) unterbrochen sind.

2. Rohrschaftinstrument nach Anspruch 1,
**dadurch gekennzeichnet, dass**
die Schneidkante (31) in einem stumpfen Winkel (β) relativ zur Schneidrichtung (X) geneigt ist.

3. Rohrschaftinstrument nach einem der vorhergehenden Ansprüche,
**dadurch gekennzeichnet, dass**
die Schneidkante (31) bogenförmig geschwungen ausgebildet ist.

4. Rohrschaftinstrument nach einem der vorhergehenden Ansprüche,
**dadurch gekennzeichnet, dass**
die Klemmflächen (11, 21) Elektroden (12, 22) zum Zuführen eines HF-Koagulationsstromes zum Gewebe umfassen.

## Claims

1. Tubular shaft instrument with a holder (40), a surgical instrument being attached to the distal end of the holder (40), the instrument comprising:
- at least one first clamping part (10) and one second clamping part (20), the first clamping part (10) having a first clamping surface (11) and the second clamping part (20) having a second clamping surface (21), the clamping parts (10, 20) being movable towards one another for gripping tissue in a closed state of the clamping surfaces (11, 21);
- a cutting device (30) with a cutting edge (31), which can move in a cutting direction in relation to the clamping parts (10, 20) to cut the gripped tissue;
- a first slit (13) in the first clamping part (10);
- a second slit (23) in the second clamping part (20), the cutting device (30) with the cutting edge (31) being movable back and forth in the slits (13) ;
the second clamping part (20) with the second clamping surface (21) being fixed on the holder (40) immovably in relation to the cutting direction;
**characterized in that** the precisely one first clamping surface (11), which is inclined at an acute angle (α) in relation to the cutting direction in the closed state, is interrupted by the first slit (13) and the precisely one second clamping surface (21), which is inclined at an acute angle (α) in relation to the cutting direction in the closed state, is interrupted by the second slit (23).

2. Tubular shaft instrument according to Claim 1, **characterized in that**
the cutting edge (31) is inclined at an obtuse angle (β) in relation to the cutting direction (X).

3. Tubular shaft instrument according to one of the preceding claims,
**characterized in that**
the cutting edge (31) is formed in an arcuately curved manner.

4. Tubular shaft instrument according to one of the preceding claims,
**characterized in that** the clamping surfaces (11, 21) comprise electrodes (12, 22) for supplying an RF coagulating current to the tissue.

## Revendications

1. Instrument à tige tubulaire comprenant un support (40), un instrument chirurgical étant fixé à l'extrémité distale du support (40), l'instrument comprenant :
- au moins une première partie de serrage (10) et une deuxième partie de serrage (20), la première partie de serrage (10) présentant une première surface de serrage (11) et la deuxième partie de serrage (20) présentant une deuxième surface de serrage (21), les parties de serrage (10, 20) pouvant être déplacées l'une par rapport à l'autre pour saisir des tissus dans un état de fermeture des surfaces de serrage (11, 21) ;
- un dispositif de coupe (30) avec une arête de coupe (31) qui peut être déplacée dans une direction de coupe par rapport aux parties de serrage (10, 20) pour couper les tissus saisis
- une première fente (13) dans la première partie de serrage (10) ;
- une deuxième fente (23) dans la deuxième partie de serrage (20), le dispositif de coupe (30) avec l'arête de coupe (31) étant déplaçable d'avant en arrière dans les fentes (13) ;
la deuxième partie de serrage (20) étant fixée avec la deuxième surface de serrage (21) par rapport à la direction de coupe de manière immobile sur le support (40) ;
**caractérisé en ce**
**que** l'exactement une première surface de serrage (11), qui est inclinée par rapport à la direction de coupe suivant un angle aigu (α) dans l'état de fermeture, est interrompue par la première fente (13) et l'exactement une deuxième surface de serrage (21), qui est inclinée par rapport à la direction de coupe suivant un angle aigu (α) dans l'état de fermeture, est interrompue par la deuxième fente (23).

2. Instrument à tige tubulaire selon la revendication 1,
**caractérisé en ce que**
l'arête de coupe (31) est inclinée par rapport à la direction de coupe (X) suivant un angle obtus (β).

3. Instrument à tige tubulaire selon l'une quelconque des revendications précédentes,
**caractérisé en ce que**
l'arête de coupe (31) est réalisée sous forme courbée en forme d'arc.

4. Instrument à tige tubulaire selon l'une quelconque des revendications précédentes,
**caractérisé en ce que**
les surfaces de serrage (11, 21) comprennent des électrodes (12, 22) pour acheminer un flux de coagulation HF aux tissus.
